# EUROPEAN PATENT APPLICATION

(11) **EP 4 563 108 A1**
(43) Date of publication of application: **04.06.2025**
(21) Application number: 24216514.0
(22) Date of filing: 29.11.2024
(51) Int. Cl.: A61B 18/12, A61B 18/20, A61N 7/00, A61N 5/06, A61B 18/00, A61N 5/00, A61B 17/00

(54) **HANDPIECE FOR TREATMENT APPARATUS**

(30) Priority: 29.11.2023 KR 20230169516
(71) Applicant: Lutronic Corporation, Goyang-si, Gyeonggi-do 10534 (KR)
(72) Inventor: LEE, Sang Hoon, 08209 Seoul (KR); LIM, Sung Jin, 10557 Gyeonggi-do (KR); JO, Min Su, 04074 Seoul (KR)
(74) Representative: Michalski Hüttermann & Partner Patentanwälte mbB

(57) **Abstract**

Disclosed is a handpiece for a treatment apparatus, which is configured to separate an energy transfer space and a refrigerant transfer space, and includes a configuration for dual insulation from a refrigerant tube.

According to the disclosure, the handpiece for the treatment apparatus is improved in convenience because medical staff does not feel cold even when cooling fluid is used in the handpiece.

In addition, the handpiece is improved in stability because heat generated from internal heating elements is efficiently removed.

## Description

### BACKGROUND OF THE INVENTION

### Field of the Invention

The disclosure relates to a handpiece, and more particularly to a handpiece capable of maintaining an appropriate temperature even during use of a treatment apparatus.

### Description of the Related Art

As a skin treatment apparatus, apparatuses that apply energy to skin tissue to denaturalize the tissue have been widely used. Due to individual differences in facial shape, treatment area, etc. among patients, different treatment methods are used to treat skin. The skin treatment apparatuses are provided with a handpiece considering the individual differences and the different treatment methods. To have an optimal treatment effect, medical staff changes a treatment position or adjusts a treatment method while holding the handpiece.

The skin treatment apparatuses widely known to date mainly heat the tissue by applying energy. Recently, the skin treatment apparatuses are provided with cooling units cool the tissue, like that of Korean Registered Patent No. 1 0-0786539 applied by the present applicant, so as to prevent the tissue from being heated to too high a temperature and permanently damaged.

Such conventional configurations for temperature change applied to the handpiece have problems of causing discomfort to medical staff who uses the handpiece while holding it with his/her hand or damaging the tissue of the medical staff.

### Documents of Related Art

(Patent Document 1) Korean Patent No. 0786539

### SUMMARY OF THE INVENTION

An aspect of the disclosure is to provide a handpiece for a treatment apparatus, which can minimize a user's discomfort due to temperature changes when using a conventional handpiece

According to the disclosure, there is provided a handpiece for a treatment apparatus including: a first housing; a second housing coupled to the first housing to define an energy transfer space on an inner side, and configured to separate a refrigerant transfer space on an outer side; a cover configured to cover the refrigerant transfer space of the second housing; an energy transfer element provided in the energy transfer space; and a refrigerant hose provided in the refrigerant transfer space.

Meanwhile, the energy transfer element may include at least one heating element that generates heat upon transferring energy.

Further, heat from the heating element may be transferred to the refrigerant transfer space through a portion of the second housing.

Meanwhile, the second housing may include a separation wall including a concave portion recessed from an outer side.

Meanwhile, the heating element may be adjacent to the separation wall.

Meanwhile, the handpiece may further include at least one rib protruding on an inner side of the cover and configured to radially support the refrigerant hose.

Meanwhile, the first housing and the second housing may be formed to extend a predetermined length, and the handpiece may further include a cap configured to couple with front end portions of the first housing and the second housing.

Meanwhile, the handpiece may further include a neck portion, which has a smaller width than other portions in an outer side formed by coupling the first housing, the second housing and the cover, so as to be held in a user's hand.

Meanwhile, the handpiece may further include a support wall provided on a rear side of the second housing and formed extending laterally.

Meanwhile, the handpiece may further include: a font connecting unit configured to support a front end portion of the refrigerant hose; and a rear connecting unit configured to support a rear end portion of the refrigerant hose.

Meanwhile, the front connecting unit may include a front end portion penetrating the cap, and a first stepped portion supported on the cap, and the rear connecting unit may include a rear end portion penetrating the support wall, and a second stepped portion protruding radially and supported on the support wall.

Meanwhile, the handpiece may further include an insulation hose provided in the refrigerant transfer space and configured to surround at least a portion of the refrigerant hose.

Meanwhile, the insulation hose may include a first end supported on the front connecting unit, and a second end supported on the rear connecting unit, and forms, together with the refrigerant hose, a first air gap in a radial direction.

In addition, the first air gap may be sealed by the refrigerant hose, the insulation hose, the front connecting unit, and the rear connecting unit.

Meanwhile, the at least one rib may be configured to support the insulation hose against the separation wall.

Further, a portion of the heating element may be configured to be in contact with the separation wall inside the energy transfer space.

Meanwhile, the concave portion of the separation wall may be configured to be in contact with an outer surface of the insulation hose.

Meanwhile, the handpiece may further include a second air gap formed between an inner surface of the cover and an outer surface of the insulation hose.

Further, the handpiece may further include a tip module including a rear side configured to couple with the cap, and a front side configured to transfer energy to skin tissue.

Meanwhile, the tip module may be detachably coupled to the cap.

### BRIEF DESCRIPTION OF THE DRAWINGS

FIG. 1 is a perspective view of a treatment apparatus with a handpiece according to an embodiment of the disclosure.
FIG. 2 is a perspective view of a handpiece for a treatment apparatus according to an embodiment of the disclosure.
FIG. 3 is a partially exploded perspective view of the handpiece of FIG. 2, from which a cover and a tip module are removed.
FIG. 4 is a cross-sectional view showing a refrigerant transfer space and an energy transfer space separately.
FIG. 5 is an exploded perspective view of a handpiece for a treatment apparatus according to an embodiment of the disclosure.
FIG. 6 is a side view of a refrigerant tube and an insulation tube according to the disclosure.
FIG. 7 is a cut-open view of a refrigerant tube and an insulation tube according to the disclosure.
FIG. 8 is a cross-sectional view taken along line I-I' in FIG. 2.
FIG. 9 is a conceptual view showing directions of thermal energy transferred through the refrigerant tube in FIG. 8.
FIG. 10 is a conceptual view showing transfer directions of thermal energy generated in a heating element of FIG. 8 toward a refrigerant transfer space.
FIG. 11 illustrates various tip modules detachably coupled to a handpiece for a treatment apparatus according to the disclosure.

### DETAILED DESCRIPTION OF THE INVENTION

Below, a handpiece for a treatment apparatus according to an embodiment of the disclosure will be described in detail with reference to the accompanying drawings. In the following description, the names of components used may be referred to as other names in this art. However, these components may be considered as equivalent components in alternative embodiments if they are functionally similar or identical to each other. Further, the reference numerals of the components are merely given for the convenience of description. However, the components indicated by the reference numerals in the accompanying drawings are not limited by those shown therein. Likewise, if components are functionally similar or identical to each other even though they are partially modified in the drawings according to alternative embodiments, the components may be considered as the equivalent components. Further, if components are recognized as components that should be included at the level of those skilled in the art, descriptions thereof will be omitted.

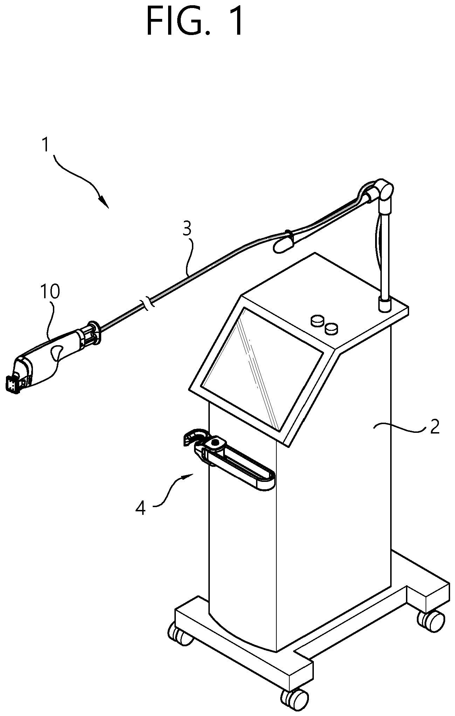

FIG. 1 is a perspective view of a treatment apparatus 1 with a handpiece 10 according to an embodiment of the disclosure.

Referring to FIG. 1, the handpiece 10 according to the disclosure may be connected to the treatment apparatus 1 and configured to perform an operation for treatment. According to the disclosure, the treatment apparatus 1 is provided with a main body 2, and may include the handpiece 10 connected to the main body 2 through a cable 3.

When the treatment apparatus 1 is a type of apparatus that produces a laser beam to perform treatment, the main body 2 may include optical elements such as a Q-switched laser (not shown), a laser oscillator (not shown), a beam distributor (not shown), a wave plate, and a secondary harmonic wave generator (not shown). Meanwhile, when the treatment apparatus is a type of apparatus that uses radio frequency (RF) energy to perform treatment, the main body 2 may be internally provided with an RF generator (not shown), an RF energy controller (not shown), a controller (not shown), etc.

Meanwhile, the foregoing types of treatment apparatus are merely examples, and the treatment apparatus may include apparatuses that transfer various types of energy, such as high-intensive focused ultrasound (HIFU).

The main body 2 may be provided with peripherals such as various input units for controlling the treatment apparatus 1, a display unit for monitoring the current state, and a handpiece holder 4 for holding the handpiece 10. The treatment apparatus 1 may typically include wheels on the bottom thereof for easy movement.

The handpiece 10 is configured so that medical staff (a user) can use the handpiece 10 while holding it with his/her by hand. The handpiece 10 is configured to receive energy generated from the main body 2 and transmit the energy to skin tissue. The handpiece 10 may be configured to receive power, an energy source, and a refrigerant from the main body 2 through the cable 3 connected to a rear side thereof. The handpiece 10 is provided with a tip module 700 at a front-end portion thereof so that the energy can be transmitted to the skin tissue through the tip module 700.

Meanwhile, an area treated by one shot of energy transmitted by the handpiece 10 is relatively small compared to the entire area required to be treated. Therefore, medical staff may repeat the treatment with several to dozens of shots of energy during skin treatment. Accordingly, medical staff often uses the handpiece 10 by holding it for a long period of time. In this case, the handpiece 10 that becomes much colder than the body temperature of the medical staff may cool his/her hand muscles, thereby making it difficult for him/her to appropriately use the handpiece 10. Likewise, the handpiece 10 that becomes too hot may also make it difficult for the medical staff to appropriately use the handpiece 10. Therefore, the handpiece 10 according to the disclosure is configured to maintain the temperature of the outer surface thereof to be held by a user at an appropriate level even when used for a long period of time.

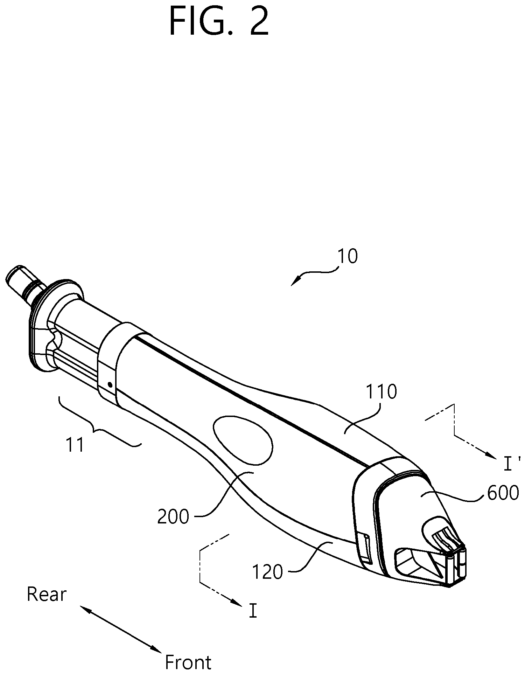

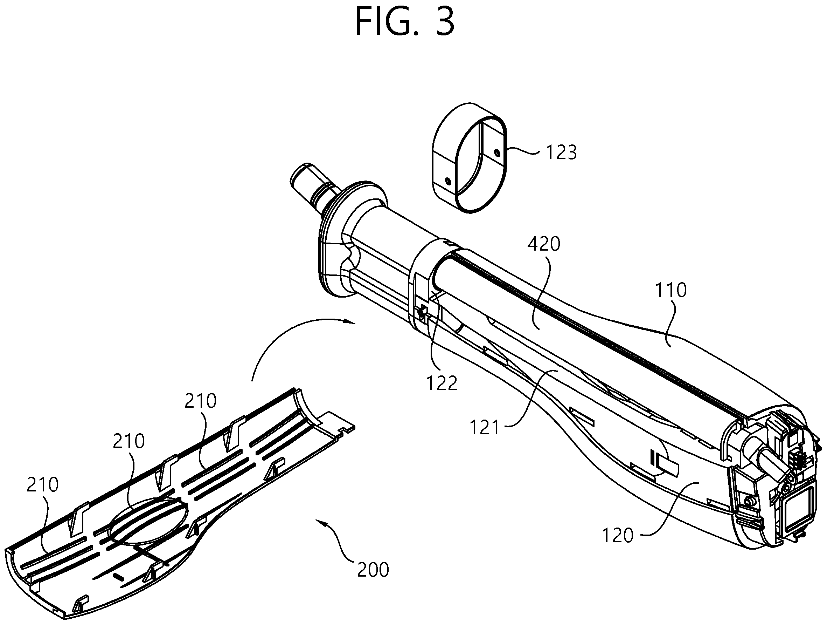

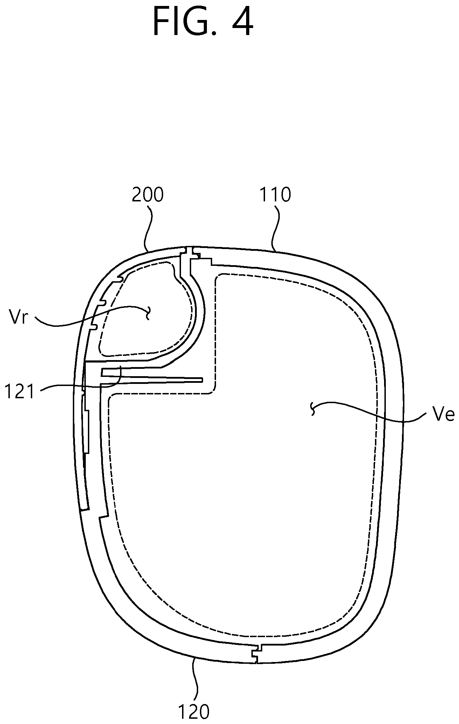

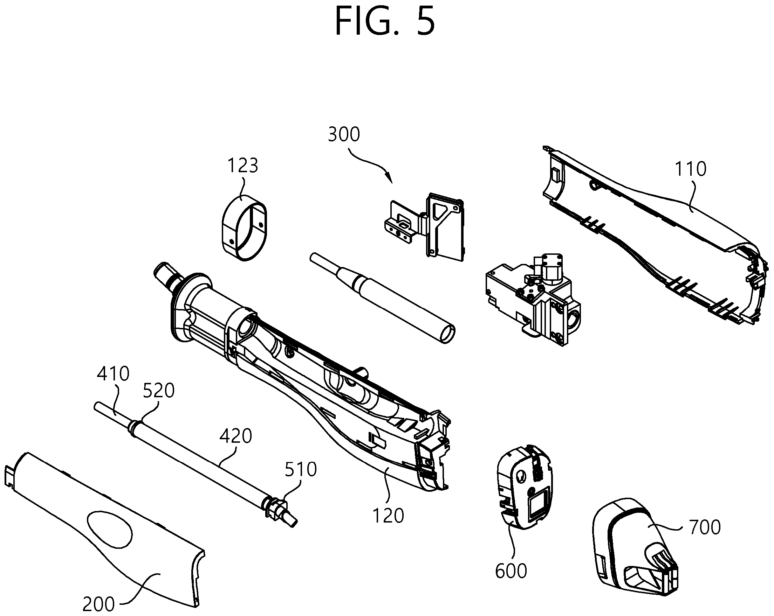

FIG. 2 is a perspective view of the handpiece 10 for the treatment apparatus according to an embodiment of the disclosure, FIG. 3 is a partially exploded perspective view of the handpiece of FIG. 2, from which a cover 200 and the tip module 700 are removed, FIG. 4 is a cross-sectional view showing a refrigerant transfer space Vr and an energy transfer space Ve separately, and FIG. 5 is an exploded perspective view of the handpiece 10 for the treatment apparatus 1 according to an embodiment of the disclosure.

Referring to FIGS. 2 to 5, the handpiece 10 according to the disclosure may include a first housing 110, a second housing 120, the cover 200, a refrigerant tube 410, an insulation tube 420, an energy transfer element 300, a cap 600, and the tip module 700.

The energy transfer space Ve to be used as a path for transferring energy inside the handpiece 10, and the refrigerant transfer space Vr to be used for cooling the tissue may be divided.

The first housing 110 and the second housing 120 may be formed to extend a predetermined length in front and rear directions. The first housing 110 and the second housing 120 are coupled to each other in a lateral direction, thereby defining the energy transfer space Ve therein. The energy transfer elements 300 may be provided inside the energy transfer space Ve. At least one of the energy transfer elements 300 may include a heating element 310 that can generate heat when transferring energy.

Meanwhile, the second housing 120 may include a separation wall 121 formed concavely inward. The separation wall 121 may be formed along the front and rear directions, and may be recessed to be large enough to allow the outer surface of the insulating tube (to be described later) to come into close contact therewith.

As the second housing 120 is formed with the recessed separation wall 121, the second housing 120 is formed asymmetrically with the first housing 110.

The cover 200 may be configured to couple with the outer side of the second housing 120. The cover 200 together with the second housing 120 defines the refrigerant transfer space Vr. The rear end portion of the cover 200 may be coupled to the second housing 120, and may be additionally locked by a locking ring 123 to prevent separation. However, this locking method is merely an example, and various coupling methods may be be applied to lock the cover 200 and maintain the refrigerant transfer space Vr.

Meanwhile, the first housing 110, the second housing 120, and the cover 200 may be coupled to form the outer appearance of the handpiece. The rear side of the handpiece 10 may partially include a neck portion 11 that has an outer circumference smaller than that of the front side. The neck portion 11 may be shaped to have the circumference gradually increasing frontwards. A user may hold the handpiece 10 in various positions according to the sizes of his/her hand. Meanwhile, a user who has small hands may use the handpiece 10 while holding the neck portion 11, which is in particular relatively small.

On the outer side of the second housing 120, a support wall 122 may be formed extending laterally from the rear side. The support wall 122 may be configured to be in contact with the rear end of the cover 200 in the lateral direction.

The refrigerant tube 410 may be used as a passage through which the refrigerant moves from the main body 2 to the tip module 700. The insulating tube is provided to surround the refrigerant tube 410. In the refrigerant transfer space Vr, the refrigerant tube may be partially provided, and the insulating tube may be entirely provided.

The refrigerant tube may be formed to a predetermined length, and have the rear end penetrating a rear connecting unit 520 and a support wall 122. The front end of the refrigerant tube 410 may be connected to a front connecting unit 510. The front connecting unit 510 includes a first insertion portion 511 on the rear side thereof, in which the front end of the refrigerant tube 410 is inserted. The front connecting unit 510 may include a nozzle 513 on the front side thereof. The front connecting unit 510 may include a first stepped portion 512 in the middle thereof as its outer diameter is expanded.

The rear connecting unit 520 includes a second insertion portion 521, the center portion of which is penetrated in the front and rear directions. The refrigerant tube may be inserted inside the second insertion portion 521. The rear connecting unit 520 may include a second stepped portion 522 on the outer side thereof as its outer diameter is expanded. The second stepped portion 522 includes a front end supported by the rear end of the insulating tube 420, and a rear end supported by the support wall 122.

The cap 600 may be configured to couple with the front end portions of the first housing 110, the second housing 120 and the cover 200 in the front and rear directions. The cap 600 may have holes formed to be penetrated by the front connecting unit 510, and at least one hole may be formed to transfer energy by the energy transfer elements. In addition, the cap 600 may be provided with an electrical connector facing frontwards and be electrically connected to the tip module 700.

The tip module 700 is configured to receive energy from the energy transfer element placed inside the handpiece 10, thereby ultimately transferring the energy to skin tissue. To this end, the front-end portion of the tip module 700 may come into at least partial contact with the skin. The tip module 700 may be detachably coupled to the front-end portion of the cap 600.

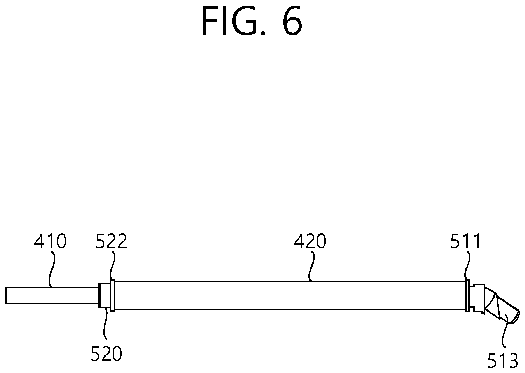

Below, the handpiece 10 for the treatment apparatus 1 according to the disclosure will be described in terms of internal thermal energy with reference to FIGS. 6 to 10. explained from the perspective of.

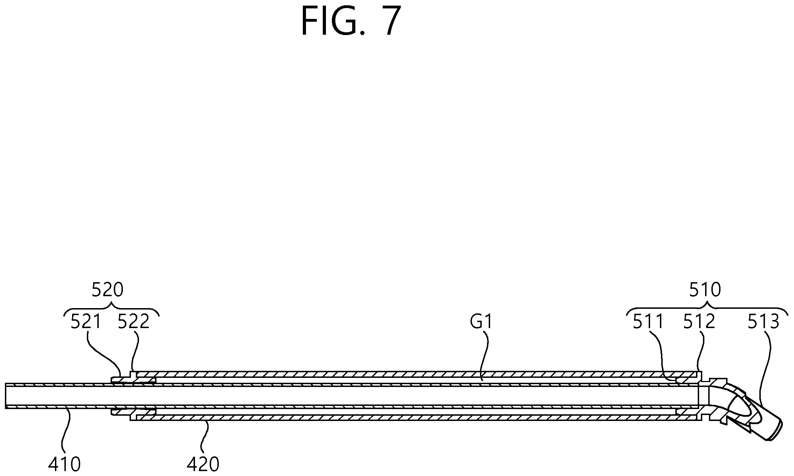

FIG. 6 is a side view of a refrigerant tube and an insulation tube according to the disclosure, and FIG. 7 is a cut-open view of the refrigerant tube 410 and the insulation tube 420 according to the disclosure.

Referring to FIGS. 6 and 7, according to the disclosure, the refrigerant transfer space Vr may be provided with the refrigerant tube 410, the insulating tube 420, a portion of the front connecting unit 510, and a portion of the rear connecting unit 520.

As described above, the front and rear ends of the refrigerant tube 410 are inserted into the front connecting unit 510 and the rear connecting unit 520, respectively. The insulating tube 420 is provided on the outer side of the refrigerant tube 410, and includes both ends supported by the first stepped portion 512 and the second stepped portion 522. In addition, the inner sides of the insulation tube 420 is supported by the outer sides of the first insertion part 511 and the second insertion part 521.

In this case, a certain gap is formed between the outer surface of the refrigerant tube 410 and the inner surface of the insulating tube 420, and will be called a first air gap G1. The first air gap G1 serves as a kind of insulating layer based on the thermal conductivity of the air itself.

Therefore, the refrigerant is maintained at a low temperature when filled in the refrigerant tube 410, and a transfer rate of thermal energy transferred by the refrigerant through the insulating tube 420 and the refrigerant tube 410 is lowered in a heat transfer space.

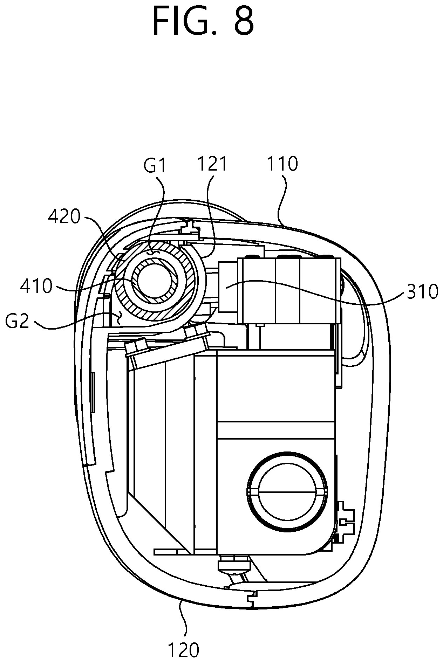

FIG. 8 is a cross-sectional view taken along line I-I' in FIG. 2.

Referring to FIG. 8, as described above, the second housing 120 is provided with the separation wall 121 recessed to form the refrigerant transfer space Vr. In locations adjacent to the separation wall 121, the refrigerant tube 410 and the heating element 310, which generates thermal energy among the energy transfer elements, may be provided.

Inside the refrigerant transfer space Vr, a dual insulation structure may be applied to the refrigerant tube 410. As described above, the insulation tube 420 primarily insulates the refrigerant tube 410.

A portion of the outer surface of the insulation tube 420 is in close contact with the separation wall 121. Another portion of the outer surface of the insulation tube 420 is in contact with a rib 210 formed to a predetermined size on the inner surface of the cover 200.

The rib 210 extends from the inner side of the cover 200 toward the refrigerant tube 410, and at least one rib 210 may be provided. The rib 210 is configured to fix the positions of the insulation tube 420 and the refrigerant tube 410 while preventing direct contact between the inner surface of the cover 200 and the insulation tube 420. The ribs 210 may support the outer surface of the insulation tube 420 at different positions. To this end, the ribs 210 may be spaced apart at a predetermined distance from each other. Therefore, a second air gap G2 may be formed between the outer surface of the insulation tube 420 and the inner surface of the cover 200 within the refrigerant transfer space Vr.

In particular, the first air gap and the second air gap may be provided inside the neck portion where a user holds the handpiece.

As described above, the dual insulation structure is applied to the refrigerant transfer space Vr and thus lowers a heat transfer efficiency as heat transfer by convection and heat transfer by conduction are alternated even though the refrigerant tube 410 is filled with the refrigerant. Therefore, a user holding the handpiece 10 does not feel cold even when the refrigerant is filled in or moves through the refrigerant tube 410.

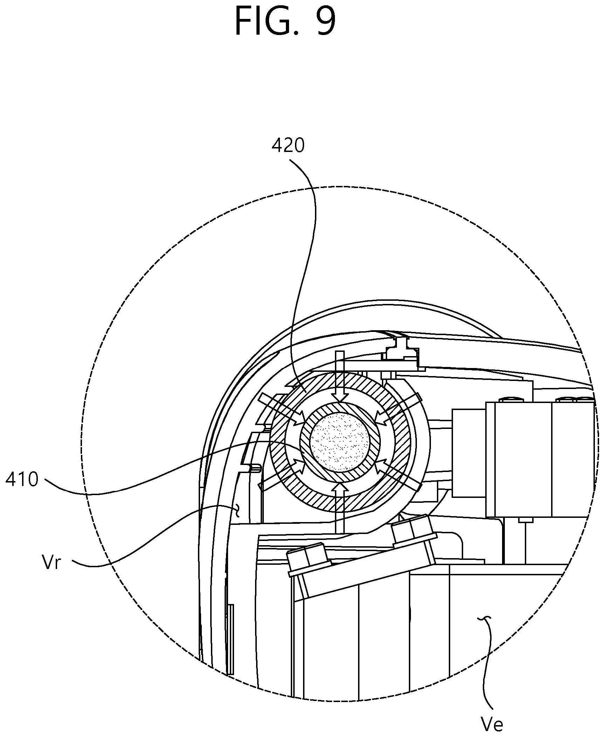

FIG. 9 is a conceptual view showing directions of thermal energy transferred through the refrigerant tube 410 in FIG. 8.

Referring to FIG. 9, the temperature of the refrigerant is lower than that of the surroundings, and thus thermal energy is transferred from the inside of the energy transfer space Ve to the refrigerant via the second air gap G2, the insulation tube 420, the first air gap G1, and the refrigerant tube 410. As described above, the refrigerant tube 410 is dually insulated from the cover 200, and thus so only a small portion of the thermal energy from the outside is transferred through the cover 200. Therefore, the amount of thermal energy loss is extremely small even when the medical staff holds the handpiece 10 with his/her hands, thereby preventing a drop in temperature.

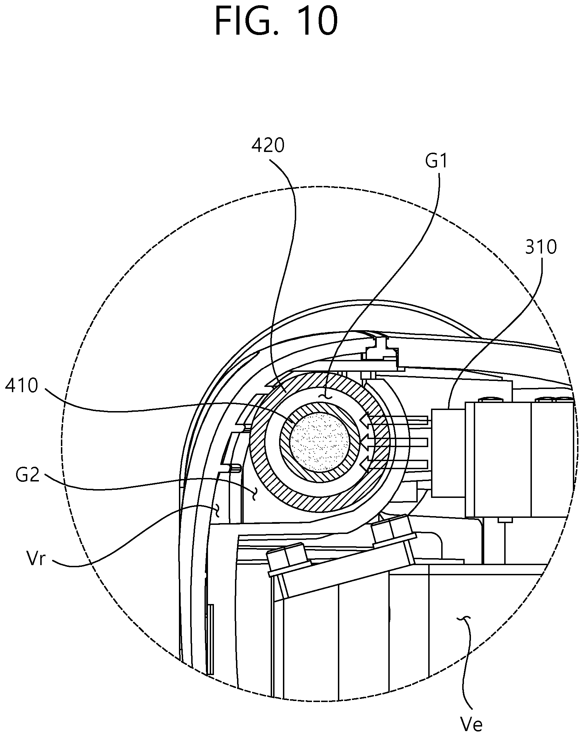

FIG. 10 is a conceptual view showing transfer directions of thermal energy generated in the heating element 310 of FIG. 8 toward a refrigerant transfer space Vr.

Referring to FIG. 10, according to the disclosure, thermal energy generated by the heating element 310 inside the energy transfer space Ve is transferred to the refrigerant transfer space Vr through the separation wall 121. In this case, the thermal energy may be transferred to the second air gap G2 and the insulation tube 420.

As described above, when the refrigerant tube 410 is full of the refrigerant, the thermal energy is continuously transferred to the refrigerant having a low temperature in the refrigerant transfer space Vr, and the temperature of the second air gap G2 is also lowered. However, the lowered temperature of the second air gap G2 may be restored to a certain extent as the thermal energy dissipated from the heating element 310 is additionally transferred to the refrigerant transfer space Vr through the separation wall 121.

Therefore, the handpiece 10 is used by a user for a long period of time without significant temperature change because the refrigerant transfer space Vr maintains a constant temperature even while the handpiece 10 operates. Further, the handpiece 10 is stably used because the heating element 310 is also easily cooled inside the handpiece 10.

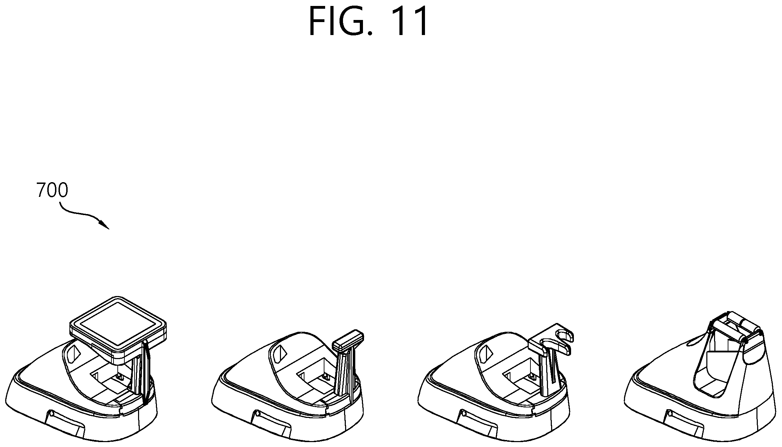

FIG. 11 illustrates various tip modules 700 detachably coupled to the handpiece 10 for a treatment apparatus 1 according to the disclosure.

Referring to FIG. 11, the tip module 700 applied to the handpiece 10 according to the disclosure may be configured in various ways depending on its purpose. Further, the tip modules 700 are configured to be detachably coupled to the cap, so that one of the tip modules 700 can be selected and mounted to the handpiece 10.

As described seen above, a handpiece for a treatment apparatus according to the disclosure is spatially partitioned into a refrigerant transfer space and an energy transfer space, and maintains a constant temperature on the outer surface thereof so that a user cannot experience discomfort. Further, the handpiece is improved in stability because temperature thereof is prevented from rising due to internal heat generation.

According to the disclosure, a handpiece for a treatment apparatus is improved in convenience because medical staff does not feel cold even when cooling fluid is used in the handpiece.

In addition, the handpiece is improved in stability because heat generated from internal heating elements is efficiently removed.

### [Reference Numerals]

| | | | |
|---|---|---|---|
| 1: | treatment apparatus | 2: | main body |
| 3: | cable | 4: | handpiece holder |
| 10: | handpiece | 11: | neck portion |
| 110: | first housing | 120: | second housing |
| 121: | separation wall | 122: | support wall |
| 200: | cover | 210: | rib |
| 300: | energy transfer element | | |
| 310: | heating element | 410: | refrigerant tube |
| 420: | insulation tube | 510: | front connecting unit |
| 511: | first insertion porti | | |
| 512: | first stepped portion | | |
| 513: | nozzle | 520: | rear connecting unit |
| 521: | second insertion portion | | |
| 522: | second stepped portion | | |
| 600: | cap | 700: | tip module |
| G1: | first air gap | G2: | second air gap |
| Ve: | energy transfer space | | |
| Vr: | refrigerant transfer space | | |

## Claims

1. A handpiece for a treatment apparatus, comprising:
a first housing;
a second housing coupled to the first housing to define an energy transfer space on an inner side, and configured to separate a refrigerant transfer space on an outer side;
a cover configured to cover the refrigerant transfer space of the second housing,
**characterized in that** an energy transfer element provided in the energy transfer space; and
a refrigerant hose provided in the refrigerant transfer space.

2. The handpiece of claim 1, wherein the energy transfer element comprises at least one heating element that generates heat upon transferring energy.

3. The handpiece of any of claims 1-2, wherein heat from the heating element is transferred to the refrigerant transfer space through a portion of the second housing.

4. The handpiece of any of claims 1-3, wherein the second housing comprises a separation wall comprising a concave portion recessed from an outer side.

5. The handpiece of any of claims 1-4, wherein the heating element is adjacent to the separation wall.

6. The handpiece of any of claims 1-5, further comprising at least one rib protruding on an inner side of the cover and configured to radially support the refrigerant hose.

7. The handpiece of any of claims 1-6, wherein
the first housing and the second housing are formed to extend a predetermined length, and
the handpiece further comprises a cap configured to couple with front end portions of the first housing and the second housing.

8. The handpiece of any of claims 1-7, further comprising a neck portion, which has a smaller width than other portions in an outer side formed by coupling the first housing, the second housing and the cover, so as to be held in a user's hand.

9. The handpiece of any of claims 1-8, further comprising a support wall provided on a rear side of the second housing and formed extending laterally.

10. The handpiece of any of claims 1-9, further comprising:
a font connecting unit configured to support a front end portion of the refrigerant hose; and
a rear connecting unit configured to support a rear end portion of the refrigerant hose.

11. The handpiece of any of claims 1-10, wherein
the front connecting unit comprises a front end portion penetrating the cap, and a first stepped portion supported on the cap, and
the rear connecting unit comprises a rear end portion penetrating the support wall, and a second stepped portion protruding radially and supported on the support wall.

12. The handpiece of any of claims 1-11, further comprising an insulation hose provided in the refrigerant transfer space and configured to surround at least a portion of the refrigerant hose.

13. The handpiece of any of claims 1-12, wherein the insulation hose comprises a first end supported on the front connecting unit, and a second end supported on the rear connecting unit, and forms, together with the refrigerant hose, a first air gap in a radial direction.

14. The handpiece of any of claims 1-13, wherein the first air gap is sealed by the refrigerant hose, the insulation hose, the front connecting unit, and the rear connecting unit.

15. The handpiece of any of claims 1-14, wherein the at least one rib is configured to support the insulation hose against the separation wall.
